# EUROPEAN PATENT APPLICATION

(11) **EP 3 903 792 A1**
(43) Date of publication of application: **03.11.2021**
(21) Application number: 20734626.3
(22) Date of filing: 06.01.2020
(51) Int. Cl.: A61K 31/734, A61P 1/12, A61P 1/14, A61P 3/02, A61P 37/02, A61P 39/06, A61P 43/00, A23L 33/21, C12N 1/20

(54) **PREBIOTIC COMPOSITION FOR BUTYRIC ACID BACTERIA**

(30) Priority: 28.12.2018 JP 2018247491
(71) Applicant: Morinaga Milk Industry Co., Ltd., Minato-ku Tokyo 108-8384 (JP)
(72) Inventor: ODAMAKI, Toshitaka, Zama-shi, Kanagawa 252-8583 (JP); HASHIKURA, Nanami, Zama-shi, Kanagawa 252-8583 (JP); MURAKAMI, Ryuta, Zama-shi, Kanagawa 252-8583 (JP); YANAGISAWA, Eiko, Zama-shi, Kanagawa 252-8583 (JP)
(74) Representative: Vossius & Partner Patentanwälte Rechtsanwälte mbB
(86) International application number: PCT/JP2020/000013
(87) International publication number: WO 2020/138511

(57) **Abstract**

A problem is to provide a prebiotic which promotes the growth of a butyrate-producing bacterium. A prebiotic composition is described that promotes the growth of a butyrate-producing bacterium which comprising alginic acid and/or a salt thereof.

SEQUENCE LISTING

<110>  Morinaga Milk Industry Co., Ltd.

<120>  Prebiotic composition for butyric acid bacteria

<130>  FP321-19412

<150>  JP2018-247491
<151>  2018-12-28

<160>  6

<170>  PatentIn version 3.5

<210>  1
<211>  32
<212>  DNA
<213>  Artificial Sequence

<220>
<223>  1st F-primer

<400>  1
cgctcttccg atctctgtac ggraggcagc ag                                    32


<210>  2
<211>  37
<212>  DNA
<213>  Artificial Sequence

<220>
<223>  1st R-primer

<400>  2
cgctcttccg atctgacgga ctachvgggt wtctaat                               37


<210>  3
<211>  73
<212>  DNA
<213>  Artificial Sequence

<220>
<223>  2nd F-primer


<220>
<221>  misc_feature
<222>  (30)..(37)
<223>  n is a, c, g, or t

<400>  3
aatgatacgg cgaccaccga gatctacacn nnnnnnnaca ctctttccct acacgacgct      60

cttccgatct ctg                                                         73


<210>  4
<211>  69
<212>  DNA
<213>  Artificial Sequence

<220>
<223>  2nd R-primer

<220>
<221>  misc_feature
<222>  (25)..(32)
<223>  n is a, c, g, or t

<400>  4
caagcagaag acggcatacg agatnnnnnn nngtgactgg agttcagacg tgtgctcttc      60

cgatctgac                                                              69

<210>  5
<211>  19
<212>  DNA
<213>  Artificial Sequence

<220>
<223>  F-primer for FP

<400>  5
ctgaggaacc tgcctcaaa                                                   19

<210>  6
<211>  19
<212>  DNA
<213>  Artificial Sequence

<220>
<223>  R-primer for FP

<400>  6
gccgtcactt ccttgttga                                                   19

## Description

### Technical Field

The present invention relates to a prebiotic composition for a butyrate-producing bacterium.

### Background Art

In recent years, relevance of enteric bacteria to the health has been drawing attention and has been studied worldwide. The term "probiotics", which is known as a term related to the improvement of enteric environment, generally refers to living microorganisms which improve the balance of the enteric flora and which thus achieve useful effects in human, and bifidobacteria and the like are generally known. "Prebiotics", which are assimilated by the probiotics, generally mean those which are not decomposed or absorbed in the upper gastrointestinal tract and which are selective nutrient sources for useful commensal bacteria in the large intestine, promote the growth thereof, improve and maintain the healthy balance of the enteric flora composition in the large intestine and serve to improve and maintain the human health.

One of the useful commensal bacteria in the large intestine is a butyrate-producing bacterium. Butyric acid is a short-chain fatty acid in the intestines and serves as the main nutrient providing energy to the large intestinal cells and also as a cell mediator which adjusts various functions not only in the intestines, such as gene expression of the host, cell differentiation, development of intestinal tissues, immunomodulation, reduction in oxidative stress and diarrhea control (NPL 1).

It is useful to take butyric acid into the body for the health. However, because butyric acid emits very strong unpleasant odor, its intake in the form of a food, a pharmaceutical product or the like is not practical. Moreover, the major butyrate-producing bacteria living in the intestines are extremely oxygen sensitive, and thus *in vitro* cultivation thereof is extremely difficult. Therefore, it is also difficult to formulate a butyrate-producing bacterium for the intake (NPL 2).

### Citation List

### Non-Patent Literature

NPL 1: Cell. 2016 Jun 2;165(6):1332-1345
NPL 2: Best. Pract. Res. Clin. Gastroenterol. 2017 Dec; 31(6):643-648.

### Summary of Invention

### Technical Problem

Considering the circumstances, the present inventors have thought that growing butyrate-producing bacteria living in the intestines is useful for maintaining and improving the health. Accordingly, a problem of the invention is to provide a prebiotic which promotes the growth of a butyrate-producing bacterium.

### Solution to Problem

As a result of intensive research to solve the problem, the inventors have found that alginic acid and/or a salt thereof can promote the growth of butyrate-producing bacteria remarkably in the intestines and reached the idea of using alginic acid and/or a salt thereof as an active ingredient in a prebiotic for promoting the growth of a butyrate-producing bacterium. The invention has been thus completed.

That is, an embodiment of the invention is a prebiotic composition for promoting the growth of a butyrate-producing bacterium comprising alginic acid and/or a salt thereof.

The butyrate-producing bacterium is preferably a bacterium of *Faecalibacterium* and/or a bacterium of *Gemmiger.*

The composition of the invention preferably comprises alginic acid and/or the salt thereof in an amount of 0.1 mass% or more of the entire composition.

The composition of the invention is preferably a food or a drink.

Moreover, the composition of the invention is preferably a pharmaceutical product.

Another embodiment of the invention is use of alginic acid and/or a salt thereof in the manufacture of a prebiotic composition for a butyrate-producing bacterium.

Another embodiment of the invention is use of alginic acid and/or a salt thereof in promoting the growth of a butyrate-producing bacterium in an intestine.

Another embodiment of the invention is alginic acid and/or a salt thereof for use in promoting the growth of a butyrate-producing bacterium in an intestine.

Another embodiment of the invention is a method for promoting the growth of a butyrate-producing bacterium in an intestine including administering alginic acid and/or a salt thereof to an animal.

In these embodiments, the butyrate-producing bacterium is preferably a bacterium of *Faecalibacterium* and/or a bacterium of *Gemmiger.*

### Advantageous Effects of Invention

According to the invention, a prebiotic which can efficiently promote the growthof a butyrate-producing bacterium in an intestine is provided. Butyric acid which the butyrate-producing bacterium produces functions as a cell mediator and maintains and improves the health. Thus, the invention is industrially extremely useful.

### Brief Description of Drawings

[Fig. 1] A graph showing the proportions of a butyrate-producing bacterium (*Faecalibacterium prausnitzii*) in all the enteric bacteria after neutralizing cultivation of the enteric bacteria with the addition of samples (n=6).
[Fig. 2] A graph showing the proportions of a butyrate-producing bacterium (*Gemmiger formicilis*) in all the enteric bacteria after neutralizing cultivation of the enteric bacteria with the addition of samples (n=6).
[Fig. 3] A graph showing the butyric acid concentrations after neutralizing cultivation of the enteric bacteria with the addition of samples (n=6).
[Fig. 4] A graph showing the bacterial counts of *Faecalibacterium prausnitzii* per 1 g feces of each subject before and after the intake of a sodium alginate material (n=6).

### Description of Embodiments

Next, the invention is explained in detail. However, the invention is not limited to the following embodiments and can be changed freely in the scope of the invention.

The composition of the invention comprises alginic acid and/or a salt thereof (also referred to as "alginic acid compounds" below).

Alginic acid is a polysaccharide comprised in marine algae such as tangle weed and *Undaria pinnatifida* and is widely used including the application to foods as a thickening and stabilizing agent. Alginic acid has a linear structure composed of β-D-mannuronic acid and α-L-guluronic acid linked by 1-4 bonds. The polymerization degree of alginic acid varies with its origin but is not particularly limited in the invention.

The salt of alginic acid is the sodium salt, the potassium salt, the calcium salt, the ammonium salt or the like and is not particularly limited in the invention, but the sodium salt and the potassium salt are preferable in view of the solubility in water.

The alginic acid compounds can be obtained by extracting from a marine alga or the like, and those commercially available may also be used for the invention.

The amount of the alginic acid compounds in the composition of the invention may be appropriately set based on the form of the composition and is not particularly limited, but for example, the amount is preferably 0.1 mass% or more of the entire composition, more preferably 1 mass% or more. The upper limit of the alginic acid compound content is not particularly restricted but is, for example, 5 mass% or less or 100 mass% or less of the entire composition. In the case of an alginate, these values are the values in terms of alginic acid. These amounts include the values during the production of the composition of the invention, the values during the distribution and the values for the intake (administration).

The composition of the invention is used as a prebiotic for a butyrate-producing bacterium. That is, the composition is assimilated by a butyrate-producing bacterium in an intestine and can promote the growth thereof.

Butyrate-producing bacterium is a generic term for the bacteria which produce butyric acid. The butyrate-producing bacterium in the invention is not particularly limited. Examples include bacteria belonging to *Faecalibacterium, Coprococcus, Clostridium, Eubacterium, Gemmiger* and the like, and more specific examples are *Faecalibacterium prausnitzii* and *Gemmiger formicilis,* which exist in the human intestines, and the like.

Here, that the composition can promote the growth of a butyrate-producing bacterium in an intestine, namely that the composition is assimilated by a butyrate-producing bacterium, can be checked by the method of Sasaki et *al.* (Sasaki K. et al., PLoS ONE 12(7): e0180991) or the following evaluation method.

Specifically, the evaluation is made by a method including a cultivation step for neutralizing cultivation of the enteric bacteria in a medium comprising the sample and an analysis step of analyzing the composition of the microbiota in the medium after the cultivation step.

When the sample to be tested (material) comprises saccharides which are generally absorbed by the human small intestine, a pretreatment step for removing the saccharides having a molecular weight of 700 or less from the sample to be tested is preferably conducted before the cultivation step. The pretreatment step is preferably conducted by ethanol precipitation. By removing the saccharides which are generally absorbed in the human small intestine, namely the saccharides having a molecular weight of 700 or less, from the sample to be tested (material) in advance through the pretreatment step and by subjecting the saccharides which are not generally absorbed in the human small intestine and which reach the large intestine to the neutralizing cultivation, the influence of the prebiotic on a butyrate-producing bacterium in the human large intestine can be evaluated accurately with high sensitivity. The saccharides having a molecular weight of 700 or less include nystose, kestose, sucrose, glucose, fructose, galactose, lactose and the like.

The neutralizing cultivation step is conducted under the conditions of pH>5.5, more preferably >6.0. The step is preferably controlled, for example, by intermittently adding an appropriate neutralizer such as sodium carbonate so that the pH preferably does not become 5.5 or less throughout the cultivation, more preferably does not become 6.0 or less. The medium used for the neutralizing cultivation is not particularly limited as long as the medium comprises substantially no saccharides which are absorbed in the small intestine, such as glucose, fructose, galactose and lactose, and an example is YCFA medium which does not comprise saccharides. Here, comprising substantially no saccharides means that the amount is preferably 0.1% (w/v) or less, more preferably 0.05% (w/v) or less, further preferably 0.01% (w/v).

The amount of the sample added to the medium can be set at any amount as long as the cultivation is not prevented, and for example, the amount can be 0.1 to 5% (w/v).

The neutralizing cultivation is generally conducted under anaerobic conditions.

The cultivation period is preferably 12 hours or longer, more preferably 24 hours or longer, and the period is generally 96 hours or shorter, more preferably 48 hours or shorter.

In the analysis step, the composition of the microbiota in the medium after the cultivation step is analyzed. For example, analysis is conducted by identifying the enteric bacteria by sequence analysis and calculating the proportions of the existing amounts. Here, when a butyrate-producing bacterium has grown through the cultivation and when the proportion of the butyrate-producing bacterium is maintained generally at 15% or more, more preferably at 30% or more, further preferably at 40% or more, the sample to be tested is determined to be a prebiotic which promotes the growth of the butyrate-producing bacterium.

The composition of the invention can be useful for a subject having a disease or a pathological condition which can be prevented or improved through an increase in butyric acid in the body or a disease or a pathological condition which is caused by a decrease in butyric acid in the body. For example, the composition can be for intestinal regulation, for immunomodulation, for reduction in oxidative stress, for prevention and/or improvement of diarrhea, for an inflammatory bowel disease, for prevention of large intestine cancer or the like.

Another embodiment of the invention is use of alginic acid and/or a salt thereof in the manufacture of a prebiotic composition for a butyrate-producing bacterium.

Another embodiment of the invention is use of alginic acid and/or a salt thereof in promoting the growth of a butyrate-producing bacterium in an intestine.

Another embodiment of the invention is alginic acid and/or a salt thereof for use in the growth of a butyrate-producing bacterium in an intestine.

Another embodiment of the invention is a method for growing a butyrate-producing bacterium in an intestine including administering alginic acid and/or a salt thereof to an animal. The animal here is not particularly limited but is generally a human.

The timing of intake (administration) of the composition of the invention is not particularly limited and can be appropriately selected depending on the condition of the subject of the administration.

The intake (dosage) of the composition of the invention is appropriately selected based on the age of the subject of the intake (administration), the gender, the condition, other conditions and the like. A standard amount of the alginic acid compounds is an amount falling in the range of preferably 1 to 300 mg/kg/day, more preferably 20 to 50 mg/kg/day.

Regardless of the amount or the period of intake (administration), the medicine can be administered once a day or in multiple divided portions.

The route of intake (administration) of the composition of the invention may be an oral or parenteral route but is generally an oral route. The parenteral intake (administration) is rectal administration or the like.

In an embodiment, the composition of the invention may comprise a butyrate-producing bacterium with the alginic acid compounds. The composition of the invention may be taken in combination with a butyrate-producing bacterium or with a preparation comprising a butyrate-producing bacterium. By the embodiment, an effect of promoting the growth of the butyrate-producing bacterium in an intestine and an effect of thus increasing butyric acid are expected to be greater.

In these embodiments, the butyrate-producing bacterium is preferably a living bacterium.

When the composition of the invention is an orally taken composition, the composition is preferably a food or a drink in an embodiment.

The form and the properties of the food or the drink are not particularly restricted as long as the food or the drink does not impair the effects of the alginic acid compounds and can be orally taken, and the food or the drink can be produced by a general method using a material which is generally used for a food or a drink except that the alginic acid compounds are added.

The food or the drink is not limited regarding the form such as liquid, paste, gel solid or powder. Examples include the following: tablet candies; liquid foods (nutrition products for tube feeding); wheat products such as breads, macaroni, spaghetti, noodles, cake mixes, frying flours and bread crumbs; instant foods such as instant noodles, cup noodles, retort-pouched/prepared foods, prepared canned foods, microwave foods, instant soups/stews, instant miso soups/clear Japanese soups, canned soups, freeze-dried foods and other instant foods; processed agricultural products such as canned agricultural products, canned fruits, jams/marmalades, pickles, cooked beans, dried agricultural products and cereals (processed grains); processed fishery products such as canned fishery products, fish hams/sausages, fishery paste products, fishery delicacies and *Tsukudani* (foods boiled down in sweetened soy sauce); processed livestock products such as canned livestock products/pastes and livestock hams/sausages; milk/dairy products such as processed milk, milk beverages, yogurts, lactic acid bacteria beverages, cheeses, ice creams, modified milk powders, creams and other dairy products; oils and fats such as butter, margarine and vegetable oils; basic condiments such as soy sauce, soybean paste, sauces, processed tomato condiments, *Mirin* (sweet sake for seasoning) and vinegars; compound flavor enhancers/foods such as cooking mixes, curry roux, sauces, dressings, noodle broths, spices and other compound flavor enhancers; frozen foods such as frozen food materials, semi-cooked frozen foods and cooked frozen foods; confectioneries such as caramels, candies, chewing gums, chocolates, cookies, biscuits, cakes, pies, snacks, crackers, Japanese-style confectioneries, rice confectioneries, bean confectioneries, desserts, jellies and other confectioneries; luxury beverages such as carbonated drinks, natural juices, fruit juices, fruit juice-comprising soft drinks, fruit flesh drinks, fruit granule-comprising fruit juices, vegetable drinks, soy milk, soy milk drinks, coffee drinks, tea drinks, drink powders, concentrated drinks, sport drinks, nutritional drinks, alcohols and other luxury beverages, other commercial foods such as baby foods, *Furikake* (dry Japanese seasonings) and seasonings for *Chazuke* (boiled rice with hot tea); infant modified milk powder; enteral nutrition products; functional foods (foods for specified health uses and foods with nutrient function claims); and the like.

An embodiment of the food or the drink can be in the form of feed. The feed is pet food, livestock feed, fish farming feed or the like.

The form of the feed is not particularly restricted and comprises, in addition to the alginic acid compounds, for example grain such as corn, wheat, barley, rye and milo; vegetable oil cake such as soybean oil cake, rapeseed oil cake, coconut oil cake and linseed oil cake; bran such as oat bran, wheat bran, rice bran and defatted rice bran; a food manufacturer's by-product such as corn gluten meal and corn jam meal; animal feed such as fish powder, defatted milk powder, whey, yellow grease and tallow; yeast such as torula yeast and brewer's yeast; mineral feed such as tertiary calcium phosphate and calcium carbonate; an oil or a fat; a single amino acid; a saccharide; or the like.

When the composition of the invention is an embodiment of a food or a drink (including feed), the composition can be provided/sold as a food or a drink labeled with use as a prebiotic for a butyrate-producing bacterium or for growing a butyrate-producing bacterium in an intestine.

The "labeling" act includes all the acts for informing a consumer of the use, and all the expressions which remind of/cause to guess the use are the "labeling" acts of the invention, regardless of the purposes of labeling, the contents of labeling, the objects to be labeled, the media and the like.

The "label" is preferably expressed so that a consumer can directly recognize the use. Specific examples include an act of transferring an article in which the use is described on a product regarding the food or the drink or packaging of a product, delivering such an article, displaying such an article for transfer or delivery or importing such an article, an act of displaying or distributing an advertisement of a product, a price list or a business document with a description of the use thereon or providing information with such contents with a description of the use by an electromagnetic method (internet or the like) and another act.

The content of the label is preferably approved by the government or the like (for example, a label approved based on a system provided by the government and provided in the form based on the approval or the like). It is preferable to label with such a content on packaging, a container, a catalogue, a brochure, an advertisement material in a sales site such as POP, other documents or the like.

The "labels" also include labels with health foods, functional foods, enteral nutrition products, food for special dietary uses, food with health claims, foods for specified health uses, foods with nutrient function claims, foods with function claims, quasi-drugs and the like. In particular, the labels are labels approved by the Consumer Affairs Agency, such as labels approved by the systems for foods for specified health uses, foods with nutrient function claims or foods with function claims and labels approved by a similar system, and the like. Specific examples include a label with foods for specified health uses, a label with qualified foods for specified health uses, a label indicating influence on the structure or the function of a body, a label with reduction of disease risk, a label with a scientifically grounded function and the like. More specifically, typical examples include labels with food for specified health uses (especially labels with health uses) provided by the Cabinet Office Ordinance on Labeling Permission for Special Dietary Uses under the Health Promotion Act (Cabinet Office Ordinance No. 57 on August 31, 2009) and similar labels.

The labels are, for example, labels with "for those who wish to increase butyrate-producing bacteria", "for those who wish to increase bacteria of *Faecalibacterium",* "intestinal regulation effect with butyric acid" and the like.

In an embodiment, the composition of the invention can be a pharmaceutical product.

The administration route of the pharmaceutical product may be an oral or parenteral route but is preferably an oral route. The parenteral intake (administration) is rectal administration or the like.

Regarding the form of the pharmaceutical product, the composition can be appropriately formulated into a desired dosage form depending on the administration method. For example, in the case of oral administration, the composition can be formulated into a solid preparation such as powder, granules, tablets and capsules, a liquid preparation such as a solution, a syrup, a suspension and an emulsion or the like. In the case of parenteral administration, the composition can be formulated into a suppository, ointment, an injection or the like.

For the formulation, in addition to the alginic acid compounds, a component which is generally used for formulation such as excipients, pH-adjusting agents, colorants and corrigents can be used. Another medicinal component, a prebiotic which is known or will be found in the future or the like can also be used in combination.

In addition, the formulation can be appropriately conducted by a known method depending on the dosage form. For the formulation, a carrier for formulation can be appropriately blended and formulated.

Examples of the excipients include: saccharide derivatives such as lactose, sucrose, glucose, mannitol and sorbitol; starch derivatives such as cornstarch, potato starch, α-starch, dextrin and carboxymethyl starch; cellulose derivatives such as crystalline cellulose, hydroxypropyl cellulose, hydroxypropyl methylcellulose, carboxymethylcellulose and carboxymethyl cellulose calcium; gum arabic; dextran; pullulan; silicate derivatives such as light silicic anhydride, synthetic aluminum silicate and magnesium aluminometasilicate; phosphate derivatives such as calcium phosphate; carbonate derivatives such as calcium carbonate; sulfate derivatives such as calcium sulfate; and the like.

Examples of binders include, in addition to the excipients: gelatin; polyvinylpyrrolidone; macrogol; and the like.

Examples of disintegrating agents include, in addition to the excipients, chemically modified starch or cellulose derivatives such as croscarmellose sodium, sodium carboxymethyl starch and cross-linked polyvinylpyrrolidone and the like.

Examples of lubricants include: talc; stearic acid; metal stearates such as calcium stearate and magnesium stearate; colloidal silica; waxes such as peegum and spermaceti wax; boric acid; glycols; carboxylic acids such as fumaric acid and adipic acid; sodium carboxylates such as sodium benzoate; sulfates such as sodium sulfate; leucine; lauryl sulfates such as sodium lauryl sulfate and magnesium lauryl sulfate; silicic acid such as silicic anhydride and silicic acid hydrate; starch derivatives; and the like.

Examples of stabilizers include: paraoxybenzoate esters such as methylparaben and propylparaben; alcohols such as chlorobutanol, benzyl alcohol and phenylethyl alcohol; benzalkonium chloride; acetic anhydride; sorbic acid; and the like.

Examples of flavoring agents include sweeteners, acidulants, aromas and the like.

In this regard, the carriers used in the case of a liquid preparation for oral administration include solvents such as water and the like.

The timing for taking the pharmaceutical product of the invention is not particularly limited, and examples include before a meal, after a meal, between meals, before bedtime and the like.

### Examples

The invention is explained more specifically below using Examples, but the invention is not limited to these Examples.

### <Test Example 1> In Vitro Examination Test of Growth of Butyrate-Producing Bacteria

### (1) Pretreatment of Samples to be Tested

The samples to be tested were prepared as follows using sodium alginate, inulin, pure cocoa and Pinefiber.

Commercial sodium alginate materials ("I-S (molecular weight of about 3000000 to 4000000)", "ULV-L3 (molecular weight of 40000 to 60000)" and "IL-6 (molecular weight of about 40000 to 60000)", each manufactured by KIMICA Corporation)) in an amount of 10 g were each dissolved in 50 mL of 99.5% ethanol (Wako Corporation 050-00446). Then, the supernatants were removed by centrifugation at 1,300×g for 10 minutes, and then 45 mL of 99.5% ethanol was added and mixed, followed by centrifugation at 1,300×g for 10 minutes. The supernatants were removed again, and the precipitates were freeze-dried. Powders were thus obtained. The powders were dissolved in MilliQ water to 0.1% (w/v) and then filtered through a 0.22 µm filter (manufactured by Merck Millipore), and solutions to be subjected to the test were thus obtained.

After dissolving 10 g of commercial inulin (manufactured by Fuji Nihon Seito Corporation) in 100 mL of MilliQ water, the solution was dispensed to centrifuge tubes (FALCON 352070) each in about 15 mL, and 30 mL of 99.5% ethanol (Wako Corporation 050-00446) was added and mixed. Then, the supernatants were removed by centrifugation at ×1,300 g for 10 minutes, and 45 mL of 99.5% ethanol was then added and mixed, followed by centrifugation at ×1,300 g for 10 minutes. The supernatants were removed again, and the precipitates were freeze-dried. Powder in a total amount of 8.7 g was thus obtained. The powder was dissolved in MilliQ water to 0.1% (w/v) and then filtered through a 0.22 µm filter (manufactured by Merck Millipore), and a solution to be subjected to the test was thus obtained.

After dissolving 400 g of commercial pure cocoa (manufactured by Morinaga & Co., Ltd.) in 2000 mL of MilliQ water, the solution was boiled for two hours. Then, the solution was dispensed to centrifuge tubes (FALCON 352070) each in about 15 mL, and 30 mL of 99.5% ethanol (Wako Corporation 050-00446) was added and mixed. Then, the supernatants were removed by centrifugation at 1,300×g for 10 minutes, and 45 mL of 99.5% ethanol was then added and mixed, followed by centrifugation at 1,300×g for 10 minutes. The supernatants were removed again, and the precipitates were freeze-dried. Powder in a total amount of 14.3 g was thus obtained. The powder was dissolved in MilliQ water to 0.1% (w/v) and then filtered through a 0.22 µm filter (manufactured by Merck Millipore), and a solution to be subjected to the test was thus obtained.

Pinefiber (manufactured by Matsutani Chemical Industry Co., Ltd.), which is indigestible dextrin, is not absorbed in the small intestine and thus was subjected to the neutralizing cultivation below without any pretreatment.

### (2) Neutralizing Cultivation

YCFA medium comprising no saccharides in an amount of 100 mL was produced and put into vessels of a pH-controllable fermenter Bio Jr.8 (BJR-25NA1S-8M manufactured by Biott Corporation), and 1 g of the samples to be tested pretreated in (1) were added. As a negative control, a medium to which the samples to be tested were not added was also prepared. The media in the vessels were autoclaved at 115°C for 20 minutes. Then, a vitamin solution and a cysteine solution which were sterilized by filtration were aseptically added, and culture solutions having a final concentration of the sample to be tested of 1% (w/v) were thus prepared. Then, the vessels were brought to anaerobic conditions through overnight nitrogen substitution, and 100 µL of fecal solutions which had been adjusted to 10% (w/v) in advance with physiological saline were added. The solutions were cultured anaerobically for 24 hours at 37°C while the pH was controlled so to not become 6 or less with 1M Na₂CO₃ solution.

The feces were provided by healthy humans (one male individual in the forties, two male individuals in the thirties, two male individuals in the twenties and one female individual in the thirties) who had had a normal diet continuously.

### (3) Analysis of Enteric Bacteria

One-milliliter samples were taken from the culture solutions after the 24-hour cultivation in (2) above and centrifuged at 15,000×g for 10 minutes, and the precipitates were obtained. The precipitates were suspended in 450 µL of an extraction solution (100 mM Tris/HCl, 4 mM EDTA, pH9.0) and then mixed with 50 µL of 10% SDS solution, 300 mg of glass beads having a diameter of 0.1 mm and 500 µL of TE saturated phenol (Wako Pure Chemical Industries, Ltd.), and the mixtures were subjected to pulverization treatment using FastPrep FP 100A (manufactured by Funakoshi Co., Ltd.) at power level 5 for 30 seconds. Next, after centrifugation at 14,000×g for five minutes, 400 µL of the supernatants were taken, and 250 µL of phenol·chloroform solution (Wako Pure Chemical Industries, Ltd.) was added and mixed. After centrifugation at 14,000×g for five minutes, 250 µL of the supernatants were obtained. The precipitates obtained by adding 250 µL of 2-propanol were dissolved in 200 µL of Tris-EDTA buffer (pH8.0) and used as template DNA solutions.

Next, a 1st primer set for amplifying the third and fourth variable regions of 16S rRNA gene of bacteria (SEQ ID NOs: 1 and 2) and a 2nd primer set which is necessary for the analysis with a next-generation sequencer Miseq (manufactured by Illumina, Inc.) (SEQ ID NOs: 3 and 4, where "n" is any nucleotide sequence that can be used for treating multiple samples in one analysis (index region)) were designed, and the primers were synthesized by the oligo primer production service of Life Technologies.

Reaction solutions comprising the template DNA solutions and the 1st primer set having a total liquid volume of 25 µL were prepared using TaKaRa Ex Taq HS kit (manufactured by Takara Bio Inc.). PCR reaction of at 94°C for three minutes and then 20 cycles at 94°C for 30 seconds, at 50°C for 30 seconds and at 72°C for 30 seconds, followed by at 72°C for 10 minutes, was conducted using Veriti 200 (manufactured by Life Technologies). The obtained PCR products were subjected to electrophoresis with 1% agarose gel, and the band patterns were examined. Subsequently, using 1 µL of the obtained PCR products as templates, PCR was conducted under the same conditions as those described above using the 2nd primer set. Here, however, the number of PCR cycles was 15 instead of 20. The obtained PCR products were subjected to electrophoresis with 1% agarose gel, and the band patterns were examined. Then, the PCR products were purified with QIAquick 96 PCR Purification Kit (manufactured by Qiagen), and the concentrations were measured with Quant-iT PicoGreen dsDNA Assay kit (manufactured by Life Technologies). Mixtures with the DNA solutions at the same concentrations were subjected to Miseq v2 Reagent kit (manufactured by Illumina, Inc.), and the sequences were analyzed by Miseq.

The compositions of the enteric microbiota were analyzed by QIIME software (version 2.0) (qiime.org) using the obtained paired-end sequences, and the proportions of the dominant butyrate-producing bacterium (*Faecalibacterium prausnitzii*) and another butyrate-producing bacterium (*Gemmiger formicilis*) in all the enteric bacteria were calculated, and the averages of all the subjects were determined.

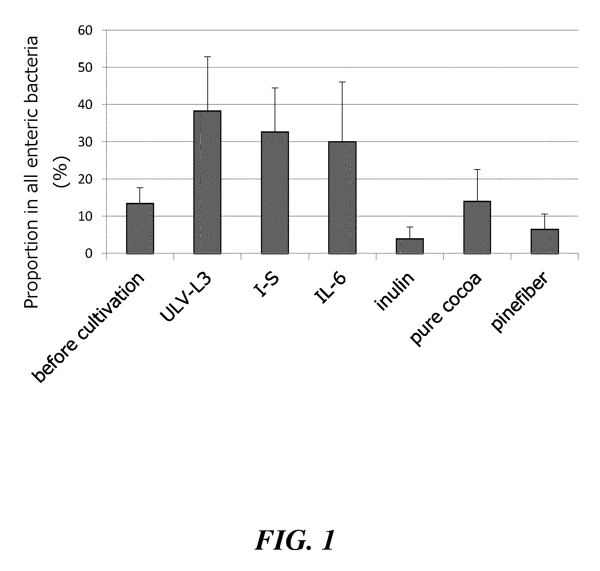

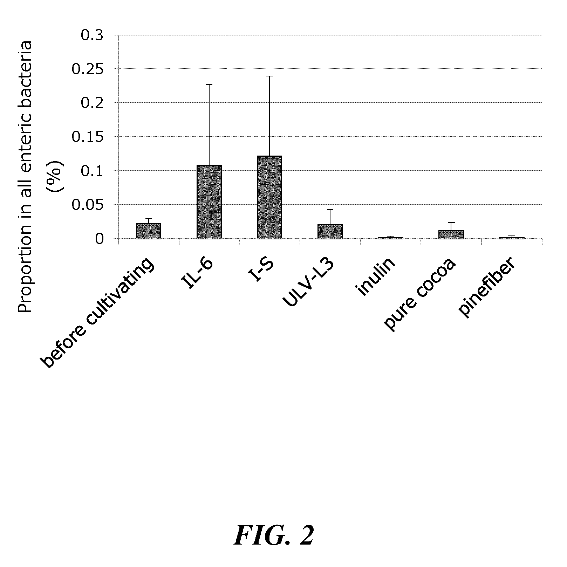

The proportions of *Faecalibacterium prausnitzii* in all the enteric bacteria are shown in Fig. 1. The proportions of *Gemmiger formicilis* in all the enteric bacteria are shown in Fig. 2. Growth of the butyrate-producing bacteria was observed in the presence of sodium alginate, compared to the results in the presence of inulin, which has been reported to increase a known butyrate-producing bacterium, or pure cocoa, which has a similar composition to that of high cocoa chocolate.

### (4) Measurement of Butyric Acid Concentrations

Using 40 µL of the culture supernatants after the 24-hour cultivation in (2) above, the following measurement was conducted.

The samples were labeled using a labeling reagent FA kit (YMC), filtered using Nano Filter Plunger w (THOMSON) and subjected to HPLC measurement.

For the measurement of butyric acid, alliance HPLC (waters) and YMC-Pack FA column (YMC) were used. Methanol was used for mobile phase A, and an aqueous 0.1% trifluoroacetic acid (TFA) solution was used for mobile phase C. An acetonitrile solution comprising 0.1% TFA dissolved therein was used for mobile phase D. The gradient program was programmed in five stages of (i) 0 to 5 minutes with mobile phase A 16%, mobile phase C 79% and mobile phase D 5%, (ii) 5 to 30 minutes with mobile phase A 16%, mobile phase C 54% and mobile phase D 30%, (iii) 30 to 35 minutes with mobile phase A 16%, mobile phase C 79% and mobile phase D 30%, (iv) 35 to 40 minutes with mobile phase A 16%, mobile phase C 79% and mobile phase D 5% and (v) 40 to 45 minutes with mobile phase A 16%, mobile phase C 79% and mobile phase D 5%. The flow rate was set at 1.00 mL/min, and the column oven temperature was set at 50°C. The measurement was made at absorbance 230 nm using a fluorescence detector.

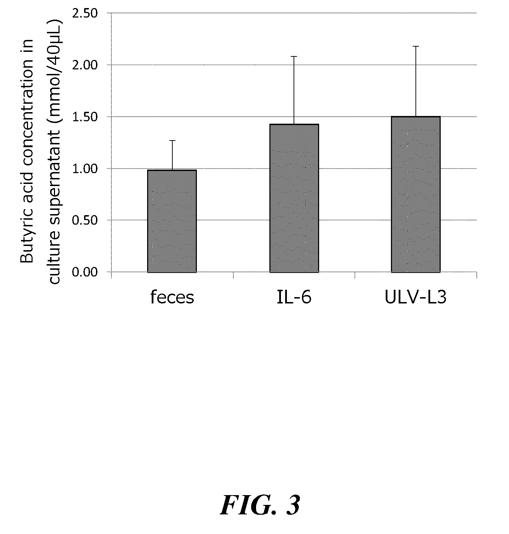

The results of the measurement of the butyric acid concentrations are shown in Fig. 3. Increases in the butyric acid concentrations were observed in the culture solutions after the neutralizing cultivation with the addition of sodium alginate (IL-6 and ULV-L3), as compared to those of the inoculated feces.

### <Test Example 2> Examination Test of Growth of Butyrate-Producing Bacterium in Humans

### (1) Intake of Sodium Alginate

Six healthy adults (three male individuals and three female individuals) who had taken 2 g of a sodium alginate material (ULV-L3) per day for five days provided an adequate amount of feces at 10°C or lower while keeping the anaerobic conditions before starting the intake and five days after the intake.

### (2) Analysis of Enteric Bacteria

Twenty milligrams of the feces provided were weighed, each suspended in 450 µL of an extraction solution (100 mM Tris/HCl, 4 mM EDTA, pH9.0) and then mixed with 50 µL of 10% SDS solution, 300 mg of glass beads having a diameter of 0.1 mm and 500 µL of TE saturated phenol (Wako Pure Chemical Industries, Ltd.), and the mixtures were subjected to pulverization treatment using FastPrep FP 100A (manufactured by Funakoshi Co., Ltd.) at power level 5 for 30 seconds. Next, after centrifugation at 14,000×g for five minutes, 400 µL of the supernatants were taken, and 250 µL of phenol·chloroform solution (Wako Pure Chemical Industries, Ltd.) was added and mixed. After centrifugation at 14,000×g for five minutes, 250 µL of the supernatants were obtained. The precipitates obtained by adding 250 µL of 2-propanol were dissolved in 200 µL of Tris-EDTA buffer (pH8.0) and used as template DNA solutions.

Reaction solutions comprising the template DNA solutions and a primer set for *Faecalibacterium prausnitzii* detection (SEQ ID NOs: 5 and 6) having a total liquid volume of 20 µL were prepared using TB Green Premix Ex Taq (manufactured by Takara Bio Inc.). Quantitative PCR reaction of at 95°C for 10 seconds, then at 95°C for three seconds and at 60°C for 30 seconds was conducted using an Applied Biosystems 7500 real-time PCR system (manufactured by Thermo Fisher), and the bacterial counts of *Faecalibacterium prausnitzii* were measured.

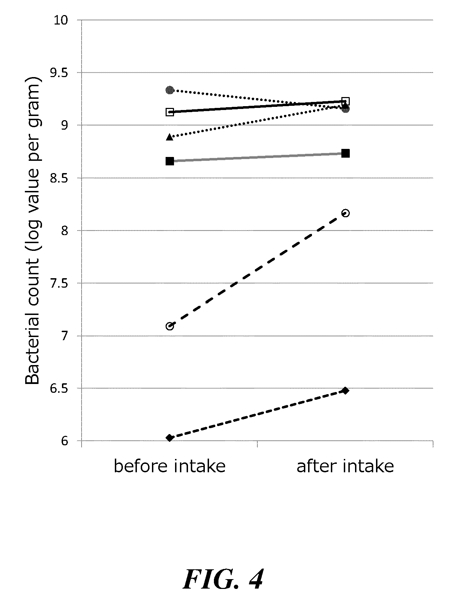

The changes in the bacterial counts of *Faecalibacterium prausnitzii* per 1 g of the feces of the respective subjects before and after starting the intake of ULV-L3 are shown in Fig. 4. Increases in *Faecalibacterium prausnitzii* were observed in five of the six subjects due to the intake of ULV-L3.

## Claims

1. A prebiotic composition for a butyrate-producing bacterium comprising alginic acid and/or a salt thereof.

2. The composition according to claim 1, wherein the butyrate-producing bacterium is a bacterium of *Faecalibacterium* and/or a bacterium of *Gemmiger.*

3. The composition according to claim 1 or 2 which comprises alginic acid and/or the salt thereof in an amount of 0.1 mass% or more of the entire composition.

4. The composition according to any one of claims 1 to 3 which is a food or a drink.

5. The composition according to any one of claims 1 to 3 which is a pharmaceutical product.

6. Use of alginic acid and/or a salt thereof in the manufacture of a prebiotic composition for a butyrate-producing bacterium.

7. The use according to claim 6, wherein the butyrate-producing bacterium is a bacterium of *Faecalibacterium* and/or a bacterium of *Gemmiger.*

8. Use of alginic acid and/or a salt thereof in the growth of a butyrate-producing bacterium in an intestine.

9. The use according to claim 8, wherein the butyrate-producing bacterium is a bacterium of *Faecalibacterium* and/or a bacterium of *Gemmiger.*

10. Alginic acid and/or a salt thereof for use in the growth of a butyrate-producing bacterium in an intestine.

11. The alginic acid and/or the salt thereof according to claim 10, wherein the butyrate-producing bacterium is a bacterium of *Faecalibacterium* and/or a bacterium of *Gemmiger.*

12. A method for growing a butyrate-producing bacterium in an intestine including administering alginic acid and/or a salt thereof to an animal.

13. The method according to claim 12, wherein the butyrate-producing bacterium is a bacterium of *Faecalibacterium* and/or a bacterium of *Gemmiger.*
